(19) Europäisches Patentamt
European Patent Office
Office européen des brevets

(11) Veröffentlichungsnummer : **0 449 247 B1**

(12) # EUROPÄISCHE PATENTSCHRIFT

(45) Veröffentlichungstag der Patentschrift :
**20.07.94 Patentblatt 94/29**

(51) Int. Cl.[5] : **A61K 31/55**

(21) Anmeldenummer : **91104858.5**

(22) Anmeldetag : **27.03.91**

(54) **Galanthemin enthaltende pharmazeutische Formulierung zur Behandlung des Alkoholismus.**

(30) Priorität : **29.03.90 DE 4010079**

(43) Veröffentlichungstag der Anmeldung :
**02.10.91 Patentblatt 91/40**

(45) Bekanntmachung des Hinweises auf die Patenterteilung :
**20.07.94 Patentblatt 94/29**

(84) Benannte Vertragsstaaten :
**AT BE CH DE DK ES FR GB GR IT LI LU NL SE**

(56) Entgegenhaltungen :
**DE-A- 3 843 239**
**US-A- 4 663 318**
**ANESTHESIOLOGY, Band 58, Nr. 6, 1983, Seiten 524-526, The American SocietyofAnesthesiologists, Inc.; J. RUPREHT et al.: "The involvement of thecentralcholinergic and endorphinergic systems in the nitrous oxide withdrawal syndrome in mice**

(56) Entgegenhaltungen :
**J. CLIN. PHARMACOL., Band 21, Nr. 1, 1981, Seiten 57-60; J.S. POWERS et al.:"Case reports. Physostigmine for treatment of delirium tremens"**
**MATER. MED. POL., Band 18, Nr. 4, 1986, Seiten 249-254; A. STOJEK et al.:"Physostigmine in eyedrops decreases craving for alcohol in earlywithdrawaltreated with carbamazepine"**

(73) Patentinhaber : **LTS Lohmann Therapie-Systeme GmbH & Co. KG**
**Postfach 23 43**
**D-56513 Neuwied (DE)**
Patentinhaber : **HEFA-FRENON ARZNEIMITTEL GMBH & CO.KG**
**Am Bahnhof 1-3**
**D-59368 Werne (DE)**

(72) Erfinder : **Opitz, K., Prof. Dr.**
**Görlitzer Str. 102**
**W-4400 Münster (DE)**

(74) Vertreter : **Flaccus, Rolf-Dieter, Dr.**
**Patentanwalt**
**Sperlingsweg 32**
**D-50389 Wesseling (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung einer Galanthamin oder eines seiner pharmazeutisch verträglichen Säureadditionssalze enthaltenden pharmazeutischen Formulierung für die Herstellung eines Arzneimittels zur Minderung des zwanghaften Alkoholverlangens bei der Behandlung des chronischen Alkoholismus. Diese Wirkstoffe werden aus entsprechenden pharmazeutischen Formulierungen, die z.B. oral, transdermal oder anderweitig parenteral verabreicht werden, auf kontinuierliche und kontrollierte Weise freigesetzt.

Insbesondere stellt die vorliegende Erfindung pharmazeutische Formulierungen zur Verfügung, die zur Behandlung des Alkoholismus geeignete Verbindungen in kontrollierter Weise freisetzen.

Während der akute Entzug bei Alkoholabhängigen und die Behandlung des lebensgefährlichen Entzugdelirs in Spezialabteilungen heute kein medizinisches Problem mehr darstellen, gibt es noch immer keine befriedigende Behandlung des chronischen Alkoholismus. Etwa 80% der therapierten Alkoholiker werden innerhalb eines Jahres rückfällig. Sie bedürfen der Hilfe durch ein zuverlässig wirkendes und gut verträgliches Mittel gegen das zum Rückfall führende Alkoholverlangen.

Trotz der mit dem Problem des chronischen Alkoholismus verbundenen menschlichen Probleme und des immensen volkswirtschaftlichen Schadens stagniert die Innovation der medikamentösen Behandlung des chronischen Alkoholismus weltweit (Naranjo, C.A., Sellers, E.M., Excerpta medica, Amsterdam - New York - Oxford, 1-9, (1985) und ganz besonders in der Bundesrepublik Deutschland (Rommelspacher, H., Wanke, K., Caspari, D., Topel, H., Dtsch. Ärzteblatt 86, 2197-B2204 (1989).

Aus der US-A-4 663 318 ist eine neue Methode zur Behandlung der Alzheimerschen Krankheit mittels Galanthamin bekannt. Der Wirkstoff oder eines seiner pharmazeutisch verträglichen Säureadditionssalze werden oral oder mittels subkutaner bzw. intravenöser Injektion bzw. durch Implantation eines wirkstoffhaltigen Reservoirs verabreicht. Zur Injektion wird eine aus Galanthamin-Kristallen durch Lösung gewonnene wäßrige Suspension verwendet.
Eine orale Einnahme erfolgt ebenfalls mittels einer wäßrigen Suspension oder in Form einer Tablette bzw. einer Kapsel. Die Dosierung wird nach Maßgabe des Körpergewichts vorgenommen und in verschiedenen Dosen von jeweils 0,5-5 mg je Kilo Körpergewicht verabreicht.

Das Dokument Mater. Med. Pol., 1986, 18(4): 249-254 beschreibt die Verwendung des reversiblen Cholinesterasehemmstoffs Physostigmin zur Behandlung des zwanghaften Alkoholverlangens (craving) in Kombination mit Carbamazepin, und weist im Vergleich mit nur mit Carbamazepin behandelten Patienten einen synergistischen Effekt der beiden Wirkstoffe nach. Es wird als Hypothese angenommen, daß diese Wirkung von Physostigmin auf seiner cholinergen Eigenschaft beruht. Zudem wird auf eine weitere, zur Publikation eingereichte Studie hingewiesen, wonach auch die alleinige Gabe von Physostigmin wirksam war.
Das Dokument J. Clin. Pharmacol. 1981, 21(1): 57-60 beschreibt die Wirkung von Physostigmin zur Erleichterung des akuten Alkoholentzugs, welche ebenfalls im Zusammenhang mit der cholinergen Wirkung von Physostigmin gesehen wird.

Bei den Pharmaka, die zur Bekämpfung der Alkoholwirkungen und des Alkoholismus eingesetzt werden, müssen unterschieden werden:

1. Ernüchterungsmittel (Amethystika),
2. Mittel zur Behandlung der lebensbedrohenden Alkoholvergiftung, z.B. Naloxon (Narcanti $^{R}$), Physostigmin;
3. Pharmaka zur Erleichterung des akuten Alkoholentzugs, z.B. Neuroleptika (Neurocil $^{R}$, Melleril $^{R}$), Piracetam, Clonidin, Carbamazepin, sowie des Entzugsdelirs: Clomethiazol (Distraneurin$^{R}$);
4. Substanzen, die den Alkoholabbau auf der Stufe des Acetaldehyds blockieren und dadurch eine künstliche Alkoholunverträglichkeit erzeugen.

   Disulfiram (Antabus $^{R}$), Hauptvertreter dieser Gruppe, ist das Medikament, das am häufigsten zur Behandlung der Alkoholkrankheit eingesetzt wird. Antabus $^{R}$ ist jedoch kein Heilmittel, da diese und verwandte Substanzen weder das Alkoholverlangen mindern noch die Ursachen der Erkrankung beeinflussen (Heinz, G., Therapie-Handbuch, Alkoholismus, 2. Aufl., Urban & Schwarzenberg, München-Wien-Baltimore, 1257-1258 (1987).
5. Mittel, die das zwanghafte Alkoholverlangen (das "craving") stillen und so dem Rückfälligwerden der therapierten Alkoholiker vorbeugen sollen. Insbesondere an diesen Mitteln besteht ein dringender Bedarf, ein wirksames Medikament zur Behandlung des chronischen Alkoholismus ist jedoch noch nicht gefunden worden.

Klinische Versuche mit Fenfluramin und Bromocriptin sind nicht wiederholt und bestätigt worden (Krasner, N., Moore, M.R., Goldberg, A., Booth, K.D., Frame, A.H., Mc Laren, A.D. Brit. J. Psychiat. 128, 346-353 (1976), Borg, V., Acta psychat. scand. 68, 100-110 (1983).

Die großen Hoffnungen, die man in die Lithiumtherapie des chronischen Alkoholismus gesetzt hatte, haben sich nicht erfüllt (Gallant, D.M., Clin. Exp. Res. 9, 297-298 (1985), Dorus, W., Ostrow, D.G., Anton, R., Cushman, P., Collins, T.F., Schäfer, M. Charles, H.L. Desai, P., Hayashida, M., Malkerneker, U., Willenbring, M., Fiscella, R., Sather, M.R., J., Amer. med. Assoc. 262, 1664-1652 (1989), Naranjo, C.A. Sellers, E.M., Roack, C.A., Woodley, D.V., Sandoz-Craig, M., Sykora, K., Clin. Pharmacol. Ther. 35, 374-381 (1984).

Die besten Erfolge sind noch mit Zimelidin erzielt worden, aber dieser Serotonin-Wiederaufnahmehemmer, der unter dem Namen Normud$^{R}$ als Antidepressivum im Handel war, mußte wegen schwerwiegender Nebenwirkungen zurückgezogen werden und steht nicht mehr zur Verfügung.

Aufgabe der Erfindung ist daher die Bereitstellung eines Arzneistoffs, der durch kontinuierliche, kontrollierte Abgabe aus einer oralen, transdermalen oder anderweitig parenteralen Formulierung eine wirksame und praktische Behandlung des chronischen Alkoholismus ermöglicht, indem er das zwanghafte Verlangen nach Alkohol mindert.

Diese Aufgabe wird erfindungsgemäß gelöst durch eine Formulierung und ihre Verwendung zur Behandlung des Alkoholismus, die dadurch gekennzeichnet ist, daß sie eine wirksame Menge des Wirkstoffs Galanthamin (4a, 5, 9, 10, 11, 12-hexa-hydro-3-methoxy-11-methyl-6H-benzofuro [3a, 3, 2-ef][2] benzazepin-6-ol)

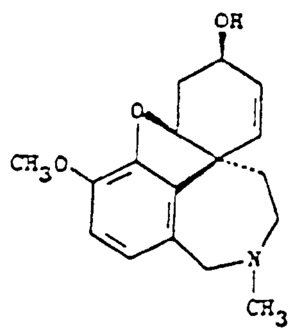

Galanthamin

oder eines seiner pharmazeutisch verträglichen Säureadditionssalze enthält.

Diese Lösung ist umso überraschender als die pharmakologischen Wirkungen des Galanthamins zwar intensiv untersucht worden sind, die auf Galanthamin zurückgehende Wirkung einer galanthaminhaltigen Formulierung, das Verlangen Alkoholkranker nach Alkohol zu mindern, aber bisher nicht beschrieben wurde.

Die Gewinnung des Galanthamins erfolgt durch Isolierung aus dem kaukasischen Schneeglöckchen Galanthus woronowii Vel., Amaryllidaceae (Proskurnina, Y., J. Gen. Chem. 22, 1899 (1952) oder durch Synthese (Kametani, T. et al, J., Chem. Soc. C., 6, 1043-1047 (1971) Shimizu, K. et al, Heterocycles 8, 277-282 (1977).

Aufgrund seiner pharmakologischen Eigenschaften gehört Galanthamin zur Gruppe der reversibel wirkenden Cholinesterasehemmstoffe. Es steht in seinen Wirkungen dem Physostigmin und dem Neostigmin nahe, besitzt jedoch auch spezifische Eigenschaften. Galanthamin hat eine 3- bis 6mal größere therapeutische Breite als Physostigmin oder Neostigmin, weil es nicht so toxisch ist (Paskov, D.S., ed. Springer-Verlag, Berlin - Heidelberg - New York - Tokio, 653-672 (1986).

Dieser Vorteil wiegt seine etwas geringere dosisbezogene Cholinesterasehemmwirkung auf.

Galanthamin wird in der Narkosepraxis zur Aufhebung der Muskelrelaxation nach nichtdepolarisierenden Muskelrelaxantien angewandt (Mayrhofer, O., Bull. schweiz. Akad. med. Wiss. 23, 48-52 (1967).

Im Gegensatz zu Neostigmin überwindet Galanthamin die Bluthirnschranke und antagonisiert die cerebrale Wirkung cholinerger Gifte. Galanthamin erweckt aus dem durch Scopolamin bewirkten Dämmerschlaf (Baraka, A., Harek, S., J. Amer. Med. Assoc. 238, 2293-2294 (1977).

Seine lange Wirkungsdauer macht Galanthamin, das die Eigenschaften von Physostigmin und Neostigmin in sich vereint, zu einem wertvollen Hilfsmittel in der Anaesthesiologie, da viele Patienten nach einer Allgemeinnarkose an einem zentralen anticholinergischen Syndrom leiden (Cozanitis, D.A., Anaesthesist 26, 649-650 (1977).

Abgesehen von seinen cholinergischen Wirkungen hat Galanthamin einen beachtlichen Einfluß auf das Atemzentrum. Die Aufhebung der durch Morphinderivate hervorgerufenen Atemdepression macht es zu einem brauchbaren Antidot für die Neuroleptanalgesie (Foitzik, H., Lawin, P., Z. prkt. Anästh. 7, 203-207 (1972), Cozanitis, D.A., Toivakka, E., J. Amer. Med. Assoc. 240, 108 (1978).

In der Neurologie wird Galanthamin bei Facialisparesen und anderen Mono- und Polyneuropathien, bei

Lähmungsrestzuständen nach Poliomyelitis oder Hirn- bzw. Rückenmarksverletzungen sowie bei der Myasthenia gravis angewandt. Bei Myasthenie soll Galanthaminhydrobromid (Nivalin R) länger wirken als Neostigmin (Prostigmin R) (Göpel, N. Bertram, N., Psychiat, Neurol. med. Psychol. 23, 712-718 (1971).

In der Augenheilkunde dient Galanthamin zur symptomatischen Behandlung des Engwinkelglaukoms (Catalino, P.U., Bolletino d'oculesta 42, 100-119 (1963), Leydhecker, W., Glaukom. Ein Handbuch, 2. Aufl., Springer-Verlag, Berlin - Heidelberg - New York, 531 (1973)).

Versuchsweise wird Galanthamin bei der Alzheimerschen Krankheit eingesetzt (Domino, E.F., Current Research in Alzheimer Therapy, Galanthamine. Another look at an old cholinesterase erihibitory E. Giacobini, R. Becker, eds. Saylor & Francies, New York - Philadelphia - Washington DC - London, 295-303 (1988).

Die vorliegende Erfindung ist auf Formulierungen gerichtet, durch die Galanthamin oder eines seiner pharmazeutisch annehmbaren Säureadditionssalze auf kontinuierliche, kontrollierte Weise abgegeben werden.

Arzneiformen, die Wirkstoffe kontrolliert freisetzen, sind im Stand der Technik bekannt. Die Verabreichung pharmazeutisch wirksamer Verbindungen mittels solcher Formulierungen kann oral, transdermal oder anderweitig parenteral erfolgen.

Die im Rahmen der vorliegenden Erfindung geeigneten Formulierungen zur oralen Verabreichung werden nachfolgend beschrieben.

In einer solchen Formulierung ist der pharmazeutische Wirkstoff in einer semipermeablen Membran eingekapselt, z.B. aus Celluloseacetat. Mit einem Bohrer oder einem Laser wird in das Kapselmaterial in winziges Loch gebohrt. Im Körper des Patienten, der behandelt wird, wird durch das Kapselmaterial Wasser absorbiert. Der pharmazeutische Wirkstoff wird durch osmotischen Druck in der gewünschten, konstanten und kontrollierten Weise allmählich durch die kleine Öffnung getrieben. Solche Systeme sind in den US-Patenten 3,760,805, 3,760,806, 3,764,984, 3,845,770, 3,916,899 und 3,987,790 beschrieben. In diesen Systemen können die pharmazeutischen Wirkstoffe in fester Form oder absorbiert an Ionenaustauscher-Harze vorliegen.

Ein anderes System zur oralen Verabreichung gemäß der vorliegenden Erfindung wird von Sheth und Leeson im US-Patent 4,137,300 beschrieben. Dieses Patent beschreibt eine Formulierung, die eine Wachsmatrix enthält.

Die Wirkstoffe der vorliegenden Erfindung werden mittels entsprechender Formulierungen auf passende und geeignete Weise verabreicht. Die festen Wirkstoffe können in Lösung oder als Dispersion verabreicht werden. Das Lösungs- oder Dispersionsmedium kann anorganisch oder organisch sein. Geeignete Lösungs- oder Suspensionsmedien für Galanthamin sind z.B. Wasser, Silikonöl oder Mineralöl.

Unter Silikonölen sollen dabei linearpolymere Dimethylsiloxane und unter Mineralölen die aus mineralischen Rohstoffen (Erdöl, Braun- und Steinkohlenteer, Holz, Torf) gewonnenen Destillationsprodukte, die im wesentlichen aus Gemischen von gesättigten Kohlenwasserstoffen bestehen, verstanden werden.

Um die Verabreichung einer Verbindung mittels einer Formulierung wie vorstehend beschrieben, zu ermöglichen, können dem System folgende Zusatzstoffe zugefügt sein:

- Antioxydantien, Synergisten, Stabilisatoren,
- Konservierungsmittel,
- Geschmackskorrigentien,
- Färbemittel,
- Lösemittel, Lösungsvermittler,
- Tenside (Emulgatoren, Solubilisatoren, Benetzer, Entschäumer),
- Viskositäts- und Konsistenzbeeinflusser, Gelbildner,
- Resorptionsbeschleuniger,
- Adsorptionsmittel,
- Feuchthaltemittel,
- Gleitmittel (z.B. Fließregulierungsmittel),
- Zerfalls- und Lösungsbeeinflusser,
- Füllmittel (Streckmittel),
- Peptisatoren,
- Freisetzungsverzögerer.

Diese Aufstellung erhebt keinen Anspruch auf Vollständigkeit. Viele Stoffe erfüllen nicht nur eine Funktion, sie sind daher mehreren der angeführten Hilfsstoffgruppen zuzuordnen. So finden z.B. Stärkearten als Füllmittel bei der Tabletten- und Pulverherstellung Verwendung. Sie sind aber zugleich Fließregulierungsmittel, Adsorptionsmittel, Hydrogelbildner und Viskositätserhöher.

Die in Frage kommenden, physiologisch unbedenklichen Substanzen sind dem Fachmann bekannt.

In einer Formulierung zur transdermalen Verabreichung von Verbindungen gemäß der vorliegenden Erfindung kann der pharmazeutische Wirkstoff in einer Matrix enthalten sein, von der er in der gewünschten allmählichen, konstanten und kontrollierten Weise abgegeben wird. Die Durchlässigkeit der Matrix bei der Freisetzung der Verbindung beruht auf Diffusion. Ein derartiges System ist in dem deutschen Patent DE 33 15 272 beschrieben. Dieses System besteht aus einer undurchlässigen Deckschicht, einem damit verbundenen, besonders aufgebauten übersättigten Wirkstoff-Reservoir aus einer Polymermatrix, einer mit dem Reservoir verbundenen, für den Wirkstoff durchlässigen Haftklebeschicht und einer die Haftklebeschicht abdeckenden, zum Gebrauch wieder ablösbaren Schutzschicht. Auch Systeme, in der die Reservoirschicht so hohe Eigenklebrigkeit aufweist, daß sie gleichzeitig die Haftklebeschicht darstellt, sind möglich.

Wenn der Wirkstoff durch die Haut absorbiert wird, erhält der zu Behandelnde auf diese Weise einen kontrollierten und vorbestimmbaren Zufluß des Wirkstoffes.

Andere geeignete transdermale Formulierungen sind in den US-Patenten 3,742,951, 3,797,494, 3,996,934 und 4,031,894 beschrieben. Diese Formulierungen bestehen grundsätzlich aus einer Rückfront, die eine der Oberflächen darstellt, einer für den Wirkstoff durchlässigen Klebschicht, die die andere Oberfläche darstellt und letztlich einem Reservoir, das den Wirkstoff zwischen den beiden die Oberflächen bildenden Schichten enthält. Alternativ dazu kann der Wirkstoff auch in einer Vielzahl von Mikrokapseln enthalten sein, die innerhalb der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut oder Mucosa des zu Behandelnden steht, kontinuierlich abgegeben. Im Falle von Mikrokapseln kann das Kapselmaterial auch als Membran wirken.

Formulierungen, die zur anderweitigen parenteralen Applikation von Galanthamin und seinen Salzen in Frage kommen, sind solche, die eine Depotwirkung des Wirkstoffes gestatten. Hierbei wird die Formulierung als Injektionslösung auf nicht-wäßriger Grundlage appliziert. Die möglichen Lösungsmittel sind dem Fachmann bekannt. Als Beispiele seien die vegetabilischen Öle erwähnt, die einzelne Pharmakopöen vorschreiben. Erdnußöl, Olivenöl, Mandelöl, Sonnenblumenöl, Sojabohnenöl und Sesamöl stehen im Vordergrund. Rizinusöl zeigt oftmals eine besonders günstige Löslichkeit für Arzneimittel; daneben sind auch Öle tierischen Ursprungs geeignet. Die Öle sind physiologisch indifferent und gut verträglich. Voraussetzung hierfür ist, daß sie besonders gereinigt sind und niedrige Säure- und Peroxidzahlen aufweisen. Da eine intravenöse Applikation wegen der fehlenden Mischbarkeit mit dem Blutserum nicht möglich ist und zur Lungenembolie führen kann, ist lediglich ihre Anwendung für intramuskuläre und subkutane Injektionspräparate möglich. Ölige Lösungen und Suspensionen verbleiben recht lange am Ort der Applikation (oft bis zu 1 Monat) und geben die Wirkstoffe protrahiert frei.

Die Dosierung des Galanthamins oder seiner pharmazeutisch annehmbaren Säureadditionssalze muß so hoch sein, daß eine nachhaltige Wirkung erzielt wird und bedarf einer individuellen Einstellung.
Der Wirkstoffgehalt der jetzigen Formulierung liegt vorzugsweise zwischen 0,1 und 90 Gew.-%, insbesondere bevorzugt zwischen 5 und 20 Gew-%, bezogen auf das Gesamtgewicht der Formulierung.

Die Erfindung wird durch das folgende Beispiel erläutert:

## Beispiel

Einfluß von Galanthamin-HBr auf den freiwilligen Alkoholkonsum von genetisch Ethanol-präferenten Ratten.

Versuchstiere waren jeweils 6 weibliche Ratten eines Stammes, der auf die finnische AA-Rattenlinie zurückgeht. Die Tiere dieses Inzuchtstammes bevorzugen 10% Alkohol als Trinkflüssigkeit, wenn ihnen reines Wasser daneben angeboten wird (free choice).

Die Tiere wurden einzeln in Makrolonkäfigen Typ 3 bei 23°C Raumtemperatur und neunmaligem Luftwechsel pro Stunde gehalten. Nur während der Dunkelphase von 20.00 Uhr bis 08.00 Uhr standen ihnen Trockenfuttergranulat (Altromin 1311; Mehl, dem Vitamine, Mineralstoffe, Aminosäuren und Spurenelemente zugesetzt sind; Hersteller: Altromin Spezialfutterwerke GmbH, 4937 Lage (Lippe)), Trinkwasser und Ethanollösung (10% v/v) in unbeschränkten Mengen zur Verfügung, und zwar in besonderen Gefäßen, die eine verlustlose Fütterung bzw. Tränkung gewährleisten. Die verbrauchten Mengen wurden gravimetrisch bestimmt, die Trinkmengen automatisch und kontinuierlich mit Hilfe von zwölf Wägezellen. Als Maß der Alkoholpräferenz diente der jeweilige Anteil Ethanollösung an der Gesamttrinkmenge in Prozent: 0% bedeutet, daß nur Wasser, keine Ethanollösung getrunken wurde, 100% bedeutet, daß ausschließlich Ethanollösung getrunken wurde. Die durchschnittliche Alkoholpräferenz der unbehandelten Versuchstiere betrug 78% bzw. 83%.
Die Ergebnisse sind in der folgenden Tabelle wiedergegeben

## Tabelle:

Einfluß von Galanthamin-hydrobromid auf das ingestive Verhalten von weiblichen Ethanol-präferenten Ratten (n=6). Die während der jeweils dreitätigen Vorperiode ohne Galanthamin-Verabreichung ermittelten

Werte stehen in runden Klammern.

| Dosis [mg/kg oral] | 5 | 10 |
|---|---|---|
| Körpergewicht.KG [g] | 215 ± 6.2 | 213 ± 4.9 |
| Alkoholpräferenz [%] | (82.8 ± 6.4)<br>45.0 ± 12.9 ** | (77.8 ± 4.3)<br>51.7 ± 6.4 *** |
| Alkoholkonsum. absolut [g/kg KG] | (6.47 ± 0.43)<br>3.17 ± 0.89** | (6.30 ± 0.34)<br>3.71 ± 0.42*** |
| Gesamttrinkmenge [g/kg KG] | (97.2 ± 2.3)<br>89.8 ± 5.6 ns | (100.9 ± 1.9)<br>90.6 ± 6.1 ns |
| Nahrungsaufnahme [g/kg KG] | (55.2 ± 1.3)<br>57.7 ± 2.6 ns | (55.7 ± 2.1)<br>44.6 ± 7.6 ns |

Student's t-Test (K. Stange, Angewandte Statistik, Springer Verlag Berlin/Heidelberg 1970) für gepaarte Werte

** $p < 0,01$

*** $p < 0,001$

ns = nicht signifikant

Wie die Tabelle zeigt, läßt sich durch Verabreichung von Galanthamin das Verlangen nach Alkohol beträchtlich reduzieren.
Nahrungsaufnahme und Gesamttrinkmenge werden nicht signifikant verändert.-

**Patentansprüche**

**Patentansprüche für folgende Vertragsstaaten : AT, BE, CH, DE, DK, FR, GB, IT, LI, LU, NL, SE**

1. Verwendung einer Galanthamin oder eines seiner pharmazeutisch verträglichen Säureadditionssalze enthaltenden pharmazeutischen Formulierung für die Herstellung eines Arzneimittels zur Minderung des zwanghaften Alkoholverlangens (craving) bei der Behandlung des chronischen Alkoholismus.

2. Verwendung nach Anspruch 1 für eine transdermale, orale oder parenterale Applikation.

3. Verwendung nach Anspruch 1 oder 2, wobei das Arzneimittel 0,1 bis 90 Gew.-%, vorzugsweise 5 bis 20 Gew.-% seines Gesamtgewichtes an Wirkstoff sowie übliche Zusatzstoffe enthält.

4. Verwendung nach einem der Ansprüche 1 bis 3, wobei das Arzneimittel eine Depotwirkung aufweist.

5. Verwendung nach einem der Ansprüche 1 bis 4, wobei der Wirkstoff in Form einer Lösung oder als Dispersion in einem anorganischen oder organischen Medium vorliegt.

6. Formulierung zur Minderung des zwanghaften Alkoholverlangens (craving) bei der Behandlung des chronischen Alkoholismus für eine kontinuierliche, kontrollierte Applikation von Galanthamin oder eines seiner pharmazeutisch verträglichen Säureadditionssalze, dadurch gekennzeichnet, daß der Wirkstoff in ein transdermales therapeutisches System eingebracht ist, bestehend aus einer undurchlässigen Rückschicht, einem damit verbundenen Wirkstoffreservoir aus einer Polymermatrix, gegebenenfalls einer die Abgabe des Wirkstoffs steuernden Membran, einer Haftklebeeinrichtung zur Befestigung des Systems auf der Haut und gegebenenfalls einer vor der Applizierung des Systems ablösbaren Schutzschicht.

7. Verfahren zur Herstellung einer Formulierung zur Minderung zwanghaften Alkoholverlangens (craving) bei der Behandlung des chronischen Alkoholismus, dadurch gekennzeichnet, daß eine wirksame Menge Galanthamin oder eines seiner pharmazeutisch vertraglichen Säureadditionssalze in fester Form oder in Form einer Lösung oder Dispersion in einem anorganischen oder organischen Medium in die Formulierung eingebracht wird, wobei übliche Zusatzstoffe zugesetzt werden können.

**Patentansprüche für folgende Vertragsstaaten : GR, ES**

1. Verfahren zur Herstellung einer Formulierung zur Minderung des zwanghaften Alkoholverlangens (craving) bei der Behandlung des chronischen Alkoholismus für eine kontinuierliche, kontrollierte Applikation von Galanthamin oder eines einer pharmazeutisch verträglichen Säureadditionssalze, dadurch gekennzeichnet, daß der Wirkstoff in ein transdermales therapeutisches System eingebracht wird, bestehend aus einer undurchlässigen Rückschicht, einem damit verbunenen Wirkstoffreservoir aus einer Polymermatrix, gegebenenfalls einer die Abgabe des Wirkstoffs steuernden Membran, einer Haftklebeeinrichtung zur Befestigung des Systtems auf der Haut und gegebenenfalls einer vor der Applizierung des Systems ablösbaren Schutzschicht.

**Claims**

**Claims for the following Contracting States : AT, BE, CH, LI, DE, DK, FR, GB, IT, LU, NL, SE**

1. The use of a pharmaceutic formulation containing galanthamine or one of the pharmaceutically acceptable acid addition salts thereof for the manufacture of a pharmaceutical product for reducing the compulsive desire (craving) for alcohol in the treatment of chronic alcoholism.

2. The use according to claim 1 for a transdermal, oral or parenteral application.

3. The use according to claim 1 or 2, whereby the pharmaceutical product contains 0.1 to 90%-wt., preferably 5 to 20%-wt., active substance, relative to the total weight of said pharmaceutical product.

4. The use according to any one of claims 1 to 3, whereby the pharmaceutical product has a depot effect.

5. The use according to any one of claims 1 to 4, whereby the active substance is present in the form of a solution or as a dispersion in an inorganic or organic medium.

6. A formulation for reducing the compulsive desire (craving) for alcohol in the treatment of chronic alcoholism for a continuous, controlled application of galanthamine or of one of the pharmaceutically acceptable acid addition salts thereof, characterized in that the active substance is incorporated in a transdermal therapeutic system consisting of an impermeable backing layer, an active substance reservoir which is made of a polymeric matrix and connected to said backing layer, optionally a membrane controlling the release of active substance, a pressure-sensitive adhesive device for fixing the system to the skin and optionally a protective layer which is removed prior to application.

7. A process for the production of a formulation for reducing the compulsive desire (craving) for alcohol in the treatment of chronic alcoholism, characterized in that an effective amount of glanthamine or one of the pharmaceutically acceptable acid addition salts thereof is incorporated in said formulation in solid form or in the form of a solution or dispersion in an inorganic or organic medium, whereby conventional adjuvants may be added.

**Claims for the following Contracting States : GR, ES**

1. A process for the production of a formulation for reducing the compulsive desire (craving) for alcohol in the treatment of chronic alcoholism, for a continuous, controlled application of galanthamine or of one of the phramaceutically acceptable acid addition salts thereof, characterized in that the active substance is incorporated in a transdermal therapeutic system consisting of an impermeable backing layer, an active substance reservoir which is made of a polymeric matrix and connected to said backing layer, optionally a membrane controlling the release of the active substance, a pressure-sensitive adhesive device for fixing the system to the skin and optionally a protective layer which is removed prior to application.

**Revendications**

**Revendications pour les Etats contractants suivants : AT, BE, CH, LI, DE, DK, FR, GB, IT, LU, NL, SE**

1. Utilisation d'une composition pharmaceutique contenant de la galanthamine ou l'un de ses sels d' addition d'acides pharmaceutiquement compatibles en vue de la préparation d'un médicament destiné à réduire le désir incoercible d'alcool (assuétude, addiction) au cours du traitement de l'alcoolisme chronique.

2. Utilisation suivant la revendication 1 convenant à l'application transdermique, orale ou parentérale.

3. Utilisation suivant la revendication 1 ou 2, caractérisée en ce que le médicament contient de 0,1 à 90% en poids, de préférence 5 à 20% en poids, par rapport à son poids total, de principe actif ainsi que des additifs usuels.

4. Utilisation suivant l'une quelconque des revendications 1 à 3, caractérisée en ce que le médicament présente un effet dépôt.

5. Utilisation suivant l'une quelconque des revendications 1 à 4, caractérisée en ce que le principe actif se présente sous la forme d'une dispersion ou d' une solution dans un milieu inorganique ou organique.

6. Composition pour réduire le désir incoercible d'alcool (addiction, assuétude) lors du traitement de l'alcoolisme chronique, destinée à une application continue et réglée de la galanthamine ou d'un de ses sels d'addition d'acides pharmaceutiquement compatibles, caractérisée en ce que le principe actif est incorporés à un système thérapeutique transdermique, qui se compose d'une couche dorsale imperméable, d'un réservoir de principe actif qui y est lié en une matrice polymérique, éventuellement d'une membrane guidant ou réglant la libération du principe actif, d'un dispositif à colle de contact pour l'assujettissement du système à la peau et éventuellement d'une couche de protection amovible avant l'application du système.

7. Procédé de préparation d'une composition pour réduire le désir incoercible d'alcool (addiction, assuétude) lors du traitement de l'alcoolisme chronique, caractérisé en ce que l'on incorpore une proportion active de galanthamine ou d'un de ses sels d'addition d'acides pharmaceutiquement compatibles, sous forme solide, ou sous la forme d'une solution ou d'une dispersion, dans un milieu inorganique ou organique dans la composition et l'on ajoute les additifs usuels.

**Revendications pour les Etats contractants suivants : GR, ES**

1. Procédé pour la préparation d'une composition pharmaceutique destinée à réduire le désir incoercible d'alcool lors du traitement de l'alcoolisme chronique et à une application continue et réglée de la galanthamine ou d'un de ses sels d'addition d'acides pharmaceutiquement compatibles, caracterisé en ce que le principe actif est incorporés à un système thérapeutique transdermique, qui se compose d'une couche dorsale imperméable, d'un réservoir de principe actif qui y est lié en une matrice polymérique, éventuellement d'une membrane guidant ou réglant la libération du principe actif, d'un dispositif à colle de contact pour l'assujettissement du système à la peau et éventuellement d'une couche de protection amovible avant l'application du système.